# EUROPÄISCHE PATENTSCHRIFT

(11) **EP 1 753 513 B1**
(45) Veröffentlichungstag und Bekanntmachung des Hinweises auf die Patenterteilung: **26.11.2008**
(21) Anmeldenummer: 05747430.6
(22) Anmeldetag: 27.04.2005
(51) Int. Cl.: A61Q 15/00, A61K 8/06, A61K 8/26, A61K 8/365

(54) **KOSMETISCHE FORMULIERUNG AUF MIKROEMULSIONSBASIS ENTHALTEND MANDELSÄURE**
COSMETIC MICROEMULSION-BASED FORMULATION CONTAINING MANDELIC ACID
PRÉPARATION COSMÉTIQUE À BASE D'UNE MICRO-ÉMULSION CONTENANT DE L'ACIDE MANDÉLIQUE

(30) Priorität: 27.04.2004 DE 102004020711; 13.04.2005 DE 102005017032
(43) Veröffentlichungstag der Anmeldung: 21.02.2007
(73) Patentinhaber: Beiersdorf Aktiengesellschaft, D-20253 Hamburg (DE)
(72) Erfinder: SCHULZ, Ulrike, 20253 Hamburg (DE); WARNKE, Katja Dr., 22547 Hamburg (DE); CHRIST, Gordon, 22763 Hamburg (DE); ROHDE, Claudia, 25499 Tangstedt (DE); DIEC, Khiet Hien, 20251 Hamburg (DE); ENGFELDT, Linda, 22047 Hamburg (DE); MIERTSCH, Heike, 22529 Hamburg (DE)
(86) Internationale Anmeldenummer: PCT/EP2005/051898
(87) Internationale Veröffentlichungsnummer: WO 2005/102256

(56) Entgegenhaltungen:
- EP-A- 0 490 053
- EP-A- 0 813 862
- EP-A- 1 092 415
- EP-A- 1 500 388
- WO-A-00/44343
- DE-A1- 19 509 079
- GB-A- 2 280 111
- US-A- 3 509 253
- US-B1- 6 180 117

## Beschreibung

Die Erfindung betrifft eine klare, kosmetische und dermatologische Formulierung auf Emulsionsbasis mit reduzierter Klebrigkeit und Fließgrenze.

Vor allem aus ästhetischen Gründen werden transparente und transluzente Produkte von vielen Verbrauchern bevorzugt. Transparente Formulierungen kommen so z. B. häufig als Deo oder Antitranspirant (AT) zum Einsatz. Diese lassen sich heutzutage durch folgende Technologien realisieren:
1. wässrig-alkoholische Formulierungen
2. Wasser-in-Silikon-Emulsionen
3. Mikro-Emulsionen

Die wässrig alkoholischen Deo- und AT-Formulierungen basieren zumeist auf Wasser und Alkohol als Medium, Deo- und Antitranspirantmittel als Wirkstoffe sowie Parfüm, Löslichkeitsvermittler und Verdicker (zumeist auf Kohlenhydratbasis) als zusätzliche Agenzien. Sie werden vom Verbraucher als frisch und kühlend empfunden, sind aber gleichzeitig mit einer ganzen Reihe an Nachteilen behaftet. So ist beispielsweise die Applikation vor allem auf frisch rasierter Haut durch den Alkoholgehalt mit Unverträglichkeiten verbunden. Ein weiterer großer Nachteil ist die Tatsache, dass in derartige Systeme keine größeren Ölmengen eingearbeitet werden können. Durch den für eine hocheffektive Wirkleistung erforderlichen hohen Gehalt an Antitranspirantsalz verbleibt nach der Applikation auf der Haut ein weißer Rückstand, der vom Verbraucher als überaus störend empfunden wird. Durch die technologisch bedingte Abwesenheit einer ausreichend großen Ölphase kann dieser allerdings nicht kaschiert werden. Darüber hinaus führt die Verwendung von Kohlenhydrat-Verdickern zu einer hohen Klebrigkeit des Produktes nach dem Verdunsten des Alkohols.

Wasser-in-Silikon-Emulsionen gehören zur Gruppe der Wasser-in-Öl-Emulsionen. Die Wasserphase, enthaltend Ethanol oder mehrwertige Alkohole wie beispielsweise Propylen Glycol und wasserlösliche Wirkstoffe wie AT-Mittel und/oder Deowirker, nimmt etwa 75-90% der Formulierung ein. Die Ölphase besteht aus einem flüchtigem und einem nicht-flüchtigen Silikonöl sowie einem Silikonemulgator.

Die Transparenz von Wasser-in-Silikon-Emulsionen basiert auf Angleichung der Brechungsindices beider Phasen. Nachteilig ist, dass schon eine z.B. durch Verdunstung bedingte Abweichung der Indices um 0,0004 zu Eintrübungen führt. WO 98/32418 und WO 92/05767 beschreiben derartige Deo- bzw. AT-Formulierungen auf W/Si-Emulsionsbasis.

Ein Ansatz zur Lösung der geschilderten Nachteile ist durch kosmetisch ansprechende alkoholfreie und transparente Produkte möglich geworden, die auf so genannten Mikroemulsionen basieren. Diese haben den großen Vorteil, dass man auch größere Mengen an verschiedenen Ölen - mit all den beschriebenen positiven Effekten für den Verbraucher - stabil einarbeiten kann. Formulierungen dieser Art sind prinzipiell mittels Phaseninversionstemperatur-Technologie (PIT) oder Hochdruckhomogenisierung zugänglich. Die notwendige Stabilität des Emulgatorsystems gegenüber hohen Konzentrationen an Antitranspirantsalzen stellt jedoch hohe Anforderungen an die Formulierungskunst des Produktentwicklers.

WO 96 28132 und WO 98 15255 beschreiben derartige Mikroemulsionen. Die Offenbarung dieser Druckschriften sind hiermit explizit Bestandteil der vorliegenden Erfindung. Nachteilig ist jedoch auch bei diesen Formulierungen ein mitunter durch den Verdicker bedingtes klebriges Hautgefühl und eine fehlende Fließgrenze der Mikroemulsion.

Die EP-A-0 490 053 offenbart ein Haarkurmittel in Form einer Mikroemulsion, umfassend ein Parfumöl sowie Zitronensäure und Wasser. Das Haarkurmittel zeichnet sich durch eine gelartige hochviskose Konsistenz aus. Die EP-A-1 092 415 offenbart Selbstbräunungssprays in Form von O/W Mikroemulsionen enthaltend Parfum und Zitronensäure. Die US-B-6,180,117 offenbart ein Verfahren zur Herstellung von kosmetischen Mikroemulsionsmischungen, wobei diese Mischungen jeweils Zitronensäure und einen Duftstoff enthalten. Die Patentanmeldung WO-A-00/44343 beschreibt kosmetische Mikroemulsionen vom Typ O/W enthaltend ebenfalls ein Parfumöl und Zitronensäure. Die EP-A-0 813 862 offenbart Parfumölkonzentrat in Form transparenter, wässriger Mikroemulsionen enthaltend zusätzlich Zitronensäure.

Aufgabe der vorliegenden Erfindung ist es, eine kosmetische Zubereitung bereit zu stellen, die den Stand der Technik bereichert und deren Nachteile vermeiden hilft. Insbesondere ist es die Aufgabe der vorliegenden Erfindung eine kosmetische und/oder dermatologische Formulierung bereit zu stellen, die transparent ist und sich durch eine minimierte Klebrigkeit auszeichnet. Insbesondere bestand die Aufgabe darin eine Deo- oder Antitranspir-antformulierung bereit zu stellen, die transparent ist und keinerlei Eintrübung aufweist, die sich durch eine minimierte Klebrigkeit auszeichnet und die eine definierte Fließgrenze zur optimierten Ausbringung und Applikation besitzt.

Gelöst wird das Bündel an Aufgaben durch eine kosmetische Formulierung entsprechend Anspruch 1. Gegenstand der Unteransprüche sind vorteilhafte Ausführungsformen der erfindungsgemäßen Zubereitung. Des weiteren umfasst die Erfindung die Verwendung derartiger Zubereitungen.

Es war überraschend und für den Fachmann nicht vorauszusehen, dass durch Zusatz von mindestens einem Antitranspirantwirkstoff, Mandelsäure und einer Mikroemulsion, insbesondere vom Typ Öl in Wasser, die Bereitstellung einer transparenten Formulierung mit definierter Fließgrenze gelingt und somit die Bereitstellung einer transparenten und wenig klebrigen kosmetischen Antitranspirant- bzw. Deodorantzubereitung ermöglicht.

Durch die überraschend einfache Kombination von Antitranspirantwirkstoffen und Mandelsäure in O/W-Mikroemulsionen lassen sich transparente kosmetische Formulierungen herstellen, die verminderte bzw. keinerlei objektiv als auch subjektiv empfundene Klebrigkeit aufweisen.

Die Hydroxyphenylessigsäure oder auch Phenylglykolsäure mit der Formel H₅C₆ -CH(OH) -COOH, C₈H₈O₃, ist bekannt unter dem Namen Mandelsäure. Die Mandelsäure ist gut löslich in Wasser, Alkohol, Ether u. 2-Propanol. Synthetisch erhält man die (±)-Mandelsäure aus Benzaldehyd und Blausäure über das α-Hydroxynitril (Cyanohydrin) und dessen saure Hydrolyse entsprechend Abbildung 1:

### Abbildung 1: Herstellung der Mandelsäure

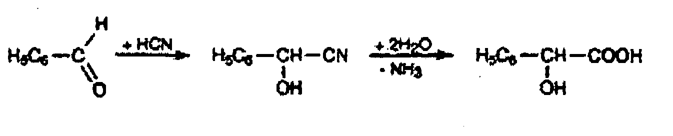

Mittels Mandelsäure, läßt sich überraschenderweise eine AT- bzw. Deodorantzubereitung herstellen, die die geforderten Eigenschaften, wie Transparenz und geringe Klebrigkeit und darüber hinaus auch die Einstellung einer bestimmten Fließgrenze der Zubereitung ermöglicht. Des weiteren zieht die erfindungsgemäße Formulierung sehr schnell ohne Rückstände zu hinterlassen in die Haut ein.

Vorteilhaft basiert die erfindungsgemäße Zubereitung auf Mikroemulsionen, bevorzugt sind O/W-Mikroemulsionen, insbesondere Mikroemulsionsgelen wie sie in der WO 98 15255 und WO 96 28132 beansprucht werden, die darin getroffenen Offenbarungen gehören hiermit explizit zum Offenbarungsgehalt der vorliegende Erfindung.

Die kosmetische Formulierung ist demnach bevorzugt auf Basis von Mikroemulsionsgelen, die a) auf Mikroemulsionen vom Typ Öl-in-Wasser beruhen, welche umfassen
- eine Ölphase, welche im wesentlichen aus schwerflüchtigen Bestandteilen zusammengesetzt ist, und eine Wasserphase
- enthaltend:
- einen oder mehrere polyethoxylierte O/W-Emulgatoren und/oder
- einen oder mehrere polypropoxylierte O/W-Emulgatoren und/oder
- einen oder mehrere polyethoxylierte und polypropoxylierte O/W-Emulgatoren,
- gewünschtenfalls ferner enthaltend einen oder mehrere W/O-Emulgatoren
- einen Emulgatorgehalt kleiner als 20 Gew.-%, bezogen auf das Gesamtgewicht der Emulsion, aufweisend,
- erhältlich auf die Weise, dass ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe auf eine Temperatur innerhalb oder oberhalb des Phaseninversionstemperaturbereiches bringt, und hernach auf Raumtemperatur abkühlt,
(b) bei welcher die Tröpfchen der diskontinuierlichen Ölphase durch eine oder mehrere Vernetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

Allerdings problematisch an den in der WO 98 15255 und WO 96 28132 beschriebenen Mikroemulsionen ist, dass eine definierte Fließgrenze nicht einstellbar war. Diese Aufgabe ist durch die vorliegende Erfindung gelöst worden.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca 1 µm bis ca. 50 µm. Solche "Makroemulsionen" sind, ohne weitere färbende Zusätze, milchig weiß gefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulich weiß gefärbt und undurchsichtig.

Mizellaren und molekularen Lösungen mit Partikeldurchmessern kleiner als ca. 10⁻² µm, ist vorbehalten, klar und transparent zu erscheinen.

Der Tröpfchendurchmesser von transparenten bzw. transluzenten Mikroemulsionen dagegen liegt im Bereich von etwa 10⁻² µm bis etwa 10⁻¹ µm. Solche Mikroemulsionen sind meist niedrigviskos. Die Viskosität vieler Mikroemulsionen vom O/W-Typ ist vergleichbar mit der des Wassers. Die Viskosität dieser Mikroemulsionen kann mit Hilfe von Assoziatiwerdickern erhöht werden, so dass dann viskose Gele vorliegen.

Die erfindungsgemäße Zubereitung liegt weiterhin vorteilhaft als Gel vor und weist eine Fließgrenze auf, wodurch die Ausbringung und Applikation gegenüber den Zubereitungen aus dem Stand der Technik verbessert ist.

Als Emulgatoren werden neben den aus dem Stand der Technik bekannten insbesondere Fettalkoholethoxylate wie beispielsweise Polyethylenglycol(16)stearylether, Fettsäureethoxylate wie beispielsweise Polyethylenglycol(14)stearat, Polyethylenglycolglycerinfettsäureester wie beispielsweise Polyethylenglycol(15)glyceryllaurat sowie als W/O-Emulgator beispielsweise Glycerylmonostearate verwendet.

Die Ölphase besteht vorzugsweise aus Estern aus gesättigten und ungesättigten, verzweigten und unverzweigten Alkancarbonsäuren oder Alkoholen mit Kettenlängen von 12-25 C-Atomen, wie beispielsweise Octyldodecanol.

Die erfindungsgemäße Kombination aus AT-Wirkstoff, Mandelsäure und Mikroemulsion, bevorzugt die in der WO 98 15255 und WO 96 28132 offenbarten Mikroemulsionen, ermöglicht über einen einzigartigen Verdickungsmechanismus die Herstelltung einer transparenten kosmetischen Zubereitung. Der Anwender hat somit erstmalig eine wasserklare und dennoch überaus wirksame Zubereitung zur Hand. Die erfindungsgemäße Zubereitung ist in Gelform bequem zu applizieren und weist ein angenehmes Hautgefühl aufgrund der fehlenden Klebrigkeit auf.

Die Kombination aus Mandelsäure, einem Antitranspirant-Wirkstoff und/oder Deodorant-Wirkstoff und eine O/W-Mikroemulsion gemäß WO98 15255 oder WO 96 28132 ermöglichen die Herstellung einer transparenten AT- oder Deodorantzubereitung, die zudem eine geringe Klebrigkeit und eine bestimmte Fließgrenze aufweist.

Als Antitranspirantwirkstoff lassen sich vorteilhaft saure Aluminiumsalze in wässriger Lösung einarbeiten. Hierbei beziehen sich die beschriebenen Konzentrationsbereiche auf die so genannten Aktivgehalte der Antitranspirant-Komplexe: bei den AluminiumVerbindungen auf wasserfreie Komplexe. Bevorzugt ist darüber hinaus auch der Einsatz von sog. aktivierten Aluminium-chlorohydraten.

Die nachfolgende Auflistung vorteilhaft einzusetzender Antitranspirant-Wirker soll in keiner Weise einschränkend sein:
Aluminium-Salze (der empirischen Summenformel [Al₂(OH)ₘClₙ], wobei m+n=6):
   - Aluminiumchlorhydrat [Al₂(OH)₅Cl] x H₂O
      Standard Al-Komplexe: Locron L, Locron LIC, Locron LIF (Clariant), Chlorhydrol (Reheis), ACH-303 (Summit), Aloxicoll L (Giulini).
      Aktivierte Al-Komplexe: Reach 501 (Reheis), Aloxicoll 51 L
   - Aluminiumsesquichlorhydrat [Al₂(OH)_{4,5}Cl_{1,5}] x H₂O
      Standard Al-Komplexe: Aloxicoll 31 L (Giulini), Westchlor 186 (Westwood Chemicals)
      Aktivierte Al-Komplexe: Reach 301 (Reheis)
   - Aluminiumdichlorhydrat [Al₂(OH)₄Cl₂] x H₂O

Die Antitranspirant-Wirkstoffe werden in den erfindungsgemäßen Formulierungen in einer Menge von 1 bis 35 Gew.%, vorzugsweise vom 1 bis 25 Gew.%, imsbesondere von 1 bis 20 Gew. %, eingesetzt.

Vorteilhaft können erfindungsgemäßen Zubereitungen auch Desodorantien zugesetzt werden. Den üblichen kosmetischen Desodorantien liegen unterschiedliche Wirkprinzipien zugrunde.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißens zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Menge der Desodorantien (eine oder mehrere Verbindungen) in den Zubereitungen beträgt vorzugsweise 0,01 bis 10 Gew.%, bevorzugt 0,05 bis 5 Gew.% bezogen auf das Gesamtgewicht der Zubereitung.

Mandelsäure wird bevorzugt in einer Menge von 0,1 bis 10 Gew.%, vorzugsweise von 0,1 bis 8 Gew.% bezogen auf die Gesamtmasse der Formulierung, eingesetzt.

Als besonders vorteilhaft hat sich eine Kombination aus Mandelsäure und Aluminium Chlorohydrat gezeigt, wobei das Verhältnis Aluminium Chlorohydrat zu Mandelsäure 15:1 bis 1:1, bevorzugt 12:1 bis 2:1, insbesondere 10:1 bis 2,5:1.

Aufgabe der vorliegenden Erfindung war es insbesondere die Sensorik der bekannten AT- bzw. Deozubereitungen zu verbessern, wie sie beispielsweise in der WO 96 28132 und WO 98 15255 beschrieben werden.

Dazu wurden folgende Vergleichsversuche auf Basis einer Mikroemulsion erstellt.

Tabelle 1 zeigt den Vergleich verschiedener transparenter Fomulierungen in einem Sensorik-Research-Panel, bestehend aus 8 geschulten Prüfern. Dazu wurden die Proben in definierter Menge auf die Haut aufgetragen und anhand einer Bewertungsskala bewertet (1 = nicht klebrig; 10 = stark klebrig).

**Tabelle 1**

| | **erfindungsgemäßes Beispiel** | **Vergleichsbeispiele** |
|---|---|---|
| | **Mikroemulsion mit Mandelsäure** | **Mikroemulsion ohne Mandelsäure, mit mehr Verdicker** |
| **Einzugsvermögen in Sekunden** | 148 | 175 |
| **Klebrigkeit Skala von 1-10** | 1,8 | 2,8 |

### Rheologietest

Die Fließgrenze oder Fließpunkt ist eine Bezeichnung für die kleinste Schubspannung, oberhalb derer ein plastischer Stoff sich rheologisch wie eine Flüssigkeit verhält (DIN 1342-1: 1983-10). Die Bestimmung der Fließgrenze erfolgt durch Aufnahme einer Fließkurve (angelehnt an die DIN 53019: 1980-05; DIN 53214: 1982-02). Der erhaltene Wert hängt stark von der Zeitskala (Belastungsrate) ab, die der Messung zugrunde liegt. Dies ist unabhängig davon, ob die Messung mit einem schubspannungs- oder drehzahlgesteuerten Viskosimeter erfolgt. Kurze Zeitskalen (schnelle Belastungen) ergeben in der Regel höhere Werte für die Fließgrenze. Eine zu hohe Fließgrenze kann Ursache von Verlaufstörungen sein. Andererseits lässt sich mit geeignet bemessener Fließgrenze die Neigung der flüssigen Formulierung zum Ablaufen unterdrücken.

Die Messungen der Fließgrenzen wurden auf einem SR-2000 Rheometer der Firma Rheometric Scientific mit folgenden Vorgaben durchgeführt:

Die Temperatur wird mit einem Peltierelement auf 25°C konstant gehalten, vor dem Test wird eine Erholungszeit von 5 Minuten abgewartet. Mit einem koaxialen aus Kunststoff bestehendem Platte/Platte Messsystem mit einem Durchmesser von 25 mm und einem Plattenabstand von 1 mm wird eine Schubspannungszeitrampe mit 40 Pa/min von 0 Pa bis 800 Pa ausgewählt. Zur Ermittlung der Fließgrenze wird die Viskosität logarithmisch über der linearen Schubspannung aufgetragen und das Viskositätsmaximum, also die kritische Schubspannung, mit der dazugehörigen maximalen Viskosität angegeben. Formulierungen ohne Fließgrenze weisen kein Maximum auf.

Formulierungen mit Fließgrenzen neigen aufgrund ihrer strukturviskosen Eigenschaft weniger zum Auslauf und eignen sich somit zur leichteren Ausbringung und Applikation. Mit Hilfe der Schubspannungszeitrampe (40 Pa/min; 25°C) konnten folgende Maxima für die Fließkurven erfindungsgemäßer Zubereitungen festgestellt werden:

| | Schubspannung (Pa) | Viskosität (Pas) |
|---|---|---|
| Mikroemulsion ohne Mandelsäure | Kein Maximum | |
| Mikroemulsion (0,7% PEG-150-Distearat) mit 1,5% Mandelsäure | 50 | 8.150 |
| Mikroemulsion (0,7% PEG-150-Distearat) mit 2,0% Mandelsäure | 111 | 96.230 |
| Mikroemulsion (1% PEG-150-Distearat) mit 1,5% Mandelsäure | 47 | 7.210 |
| Mikroemulsion (1 % PEG-150-Distearat) mit 2,0% Mandelsäure | 117 | 77.690 |

Der Vorteil der erfindungsgemäßen Mikroemulsion mit Fließgrenze ist, dass durch die Fließgrenze das Auslaufen aus dem Applikator verhindert wird, da die Formulierung nicht ohne Scherung fließfähig ist.

Die Formulierung weist bevorzugt eine definierte Fließgrenze von 40 bis 120 Pa (mittels Schubspannungszeitrampe (40 Pa/min; 25°C)) auf.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Die Herstellung der erfindungsgemäßen Zubereitung erfolgt indem Wasser- und Ölphase getrennt von einander auf 85°C erhitzt werden. Anschließen werden die Phasen unter Rühren (Blattrührer 200 U/min) vereint. Mandelsäure wird in Wasser gelöst, bei 30°C zur Emulsion gegeben und 30 min gerührt.

Zur Applikation der Zubereitung lassen sich herkömmliche Packmittel für Deodorantien und/oder Antitransipirantien verwenden, z. B. Stiftdispenser, Geldispenser, Tuben und Roller.

In den Beispielen beziehen sich die Angaben in Gewichtsprozent auf die Gesamtmasse der Zubereitung.

### Beispiele

| | 1 | 2 | 3 |
|---|---|---|---|
| Glyceryl Isostearate | 2,6 | 2,5 | 2,5 |
| Isoceteth-20 | 5 | 5 | 5 |
| PEG-150 Distearat | 1 | 1,5 | 0,7 |
| Dicaprylyl Ether | 5 | 5 | 5 |
| Mandelic Acid | 1,5 | 1,5 | 2 |
| Aluminum Chlorohydrate | 10 | 10 | 10 |
| Perfume | 1 | 1 | 1 |
| Butylene Glycol | 3 | - | 3 |
| Methylparaben | 0,2 | 0,2 | - |
| Wasser | 70,7 | 73,3 | 70,8 |

## Patentansprüche

1. Kosmetische Formulierung auf Basis einer Mikroemulsion umfassend mindestens einen Antitranspirant-Wirkstoff und/oder Deodorant-Wirkstoff sowie Mandelsäure.

2. Kosmetische Formulierung nach Anspruch 1, **dadurch gekennzeichnet, dass** es eine O/W-Mikroemulsion ist.

3. Kosmetische Formulierung nach Anspruch 1 , **dadurch gekennzeichnet, dass** es ein Mikroemulsionsgel ist.

4. Kosmetische Formulierung nach Anspruch 3 auf Basis von Mikroemulsionsgelen,
a) beruhend auf Mikroemulsionen vom Typ Öl-in-Wasser, welche umfassen
- eine Ölphase, welche im wesentlichen aus schwerflüchtigen Bestandteilen zusammengesetzt ist, und eine Wasserphase
- enthaltend:
- einen oder mehrere polyethoxylierte O/W-Emulgatoren und/oder
- einen oder mehrere polypropoxylierte O/W-Emulgatoren und/oder
- einen oder mehrere polyethoxylierte und polypropoxylierte O/W-Emulgatoren,
- gewünschtenfalls ferner enthaltend einen oder mehrere W/O-Emulgatoren
- einen Emulgatorgehalt kleiner als 20 Gew-%, bezogen auf das Gesamtgewicht der Emulsion, aufweisend,
- erhältlich auf die Weise, daß ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe auf eine Temperatur innerhalb oder oberhalb des Phaseninversionstemperaturbereiches bringt, und hernach auf Raumtemperatur abkühlt
(b) bei welcher die Tröpfchen der diskontinuierlichen Ölphase durch eine oder mehrere Vemetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

5. Formulierung nach einem der vorstehenden Ansprüche, **dadurch gekennzeichnet, dass** der Antitranspirant-Wirkstoff aus der Gruppe der Aluminium-Salze, bevorzugt Aluminium-Chlorohydrat und/oder aktiviertes Aluminium-Chlorohydrat gewählt wird.

6. Formulierung nach einem der voranstehenden Ansprüche, **dadurch gekennzeichnet, dass** das Verhältnis Alumimium chlorohydrat zu Mandelsäure im Bereich 15:1 bis 1:1, bevorzugt 12:1 bis 2:1, insbesondere 10 : 1 bis 2,5:1, gewählt wird.

7. Formulierung nach einem der voranstehenden Ansprüche, **dadurch gekennzeichnet, dass** der Antitranspirant-Wirkstoff in einer Menge von 1 bis 35 Gew.%, vorzugsweise von 1 bis 25 Gew. -%, besonders bevorzugt von 1 bis 20 Gew.% bezogen auf die Gesamtmasse der Formulierung, eingesetzt wird.

8. Formulierung nach einem der voranstehenden Ansprüche, **dadurch gekennzeichnet, dass** die Mandelsäure in einer Menge von 0,1 bis 10 Gew.%, vorzugsweise von 0,1 bis 8 Gew.% bezogen auf die Gesamtmasse der Formulierung, eingesetzt wird,

9. Formulierung nach einem der voranstehenden Ansprüche, **dadurch gekennzeichnet, dass** die Formulierung eine definierte Fließgrenze aufweist.

10. Formulierung nach Anspruch 9, **dadurch gekennzeichnet, dass** die Formulierung eine definierte Fließgrenze von 40 bis 120 Pa (mittels Schubspannungszeitrampe (40 Pa/min; 25°C)) aufweist.

11. Verwendung einer kosmetischen Formulierung nach mindestens einem der vorhergehenden Ansprüche zur Auftragung auf die menschliche Haut.

12. Verwendung einer kosmetischen Formulierung nach mindestens einem der vorhergehenden Ansprüche 1 bis 10 als Antitranspirant und/oder Deodorant.

13. Verwendung der Kombination aus Mandelsäure, einem Antitranspirant-Wirkstoff und/oder Deodorant-Wirkstoff und einer O/W-Mikroemulsion zur Herstellung einer transparenten AT- oder Deodorantzubereitung.

14. Verwendung der Kombination aus einem Antitranspirant-Wirkstoff und/oder Deodorant-Wirkstoff sowie Mandelsäure zur Herstellung einer Mikroemulsion mit definierter Fließgrenze.

## Claims

1. Cosmetic microemulsion-based formulation comprising at least one antiperspirant active ingredient and/or deodorant active ingredient and mandelic acid.

2. Cosmetic formulation according to Claim 1, **characterized in that** it is an O/W microemulsion.

3. Cosmetic formulation according to Claim 1, **characterized in that** it is a microemulsion gel.

4. Cosmetic formulation according to Claim 3 based on microemulsion gels,
a) based on microemulsions of the oil-in-water type which comprise
- an oil phase which is essentially composed of difficultly volatile constituents, and a water phase
- comprising:
- one or more polyethoxylated O/W emulsifiers and/or
- one or more polypropoxylated O/W emulsifiers and/or
- one or more polyethoxylated and polypropoxylated O/W emulsifiers,
- if desired also comprising one or more W/O emulsifiers
- having an emulsifier content of less than 20% by weight, based on the total weight of the emulsion,
- obtainable by bringing a mixture of the base components, comprising water phase, oil phase, one or more of the O/W emulsifiers according to the invention, if desired one or more W/O emulsifiers, and if desired further auxiliaries, additives and/or active ingredients, to a temperature within or above the phase inversion temperature range, and subsequently cooling to room temperature
(b) in which the droplets of the discontinuous oil phase are joined together by one or more crosslinker substances whose molecules are **characterized by** at least one hydrophilic region which has a breadth suitable for bridging the distance between the microemulsion droplets, and by at least one hydrophobic region which is able to enter into hydrophobic interaction with the microemulsion droplets.

5. Formulation according to one of the preceding claims, **characterized in that** the antiperspirant active ingredient is chosen from the group of aluminium salts, preferably aluminium chlorohydrate and/or activated aluminium chlorohydrate.

6. Formulation according to one of the preceding claims, **characterized in that** the ratio of aluminium chlorohydrate to mandelic acid is chosen in the range 15:1 to 1:1, preferably 12:1 to 2:1, in particular 10:1 to 2.5:1.

7. Formulation according to one of the preceding claims, **characterized in that** the antiperspirant active ingredient is used in an amount of from 1 to 35% by weight, preferably from 1 to 25% by weight, particularly preferably from 1 to 20% by weight, based on the total mass of the formulation.

8. Formulation according to one of the preceding claims, **characterized in that** the mandelic acid is used in an amount of from 0.1 to 10% by weight, preferably from 0.1 to 8% by weight, based on the total mass of the formulation.

9. Formulation according to one of the preceding claims, **characterized in that** the formulation has a defined yield point.

10. Formulation according to Claim 9, **characterized in that** the formulation has a defined yield point of from 40 to 120 Pa (by means of shear stress time ramp (40 Pa/min; 25°C)).

11. Use of a cosmetic formulation according to at least one of the preceding claims for application to the human skin.

12. Use of a cosmetic formulation according to at least one of the preceding Claims 1 to 10 as antiperspirant and/or deodorant.

13. Use of the combination of mandelic acid, an antiperspirant active ingredient and/or deodorant active ingredient and of an O/W microemulsion for preparing a transparent AP or deodorant preparation.

14. **U**se of the combination of an antiperspirant active ingredient and/or deodorant active ingredient and mandelic acid for preparing a microemulsion with a defined yield point.

## Revendications

1. Composition cosmétique à base d'une microémulsion comprenant au moins une substance active antisudorale et/ou une substance active déodorante ainsi que de l'acide mandélique.

2. Composition cosmétique selon la revendication 1, **caractérisée en ce qu'**elle est une microémulsion H/E.

3. Composition cosmétique selon la revendication 1, **caractérisée en ce qu'**elle est un gel de microémulsion.

4. Composition cosmétique selon la revendication 3, à base de gels de microémulsions,
a) à base de microémulsions du type huile-dans-eau qui comprennent
- une phase huileuse qui est essentiellement composée de composants peu volatils, et une phase aqueuse
- contenant :
- un ou plusieurs émulsifiants H/E polyéthoxylés et/ou
- un ou plusieurs émulsifiants H/E polypropoxylés et/ou
- un ou plusieurs émulsifiants H/E polyéthoxylés et polypropoxylés,
- éventuellement contenant en outre un ou plusieurs émulsifiants E/H
- présentant une teneur en émulsifiants inférieure à 20 % en poids, par rapport au poids total de l'émulsion,
- pouvant être obtenues en portant à une température dans ou au-dessus de à plage de température d'inversion de phase un mélange des composants de base comprenant phase aqueuse, phase huileuse, un ou plusieurs des émulsifiants H/E selon l'invention, si on le désire un ou plusieurs émulsifiants E/H, ainsi qu'éventuellement d'autres adjuvants, additifs et/ou substances actives, et ensuite en le refroidissant jusqu'à la température ambiante,
b) dans laquelle les gouttelettes de la phase huileuse discontinue sont liées les unes aux autres par une ou plusieurs substances de réticulation, dont les molécules se distinguent par au moins une région hydrophile qui présente une extension qui est appropriée à ponter la distance séparant les unes des autres les gouttelettes de la microémulsion, et par au moins une région hydrophobe qui permet d'entrer en interaction hydrophobe avec les gouttelettes de la microémulsion.

5. Composition selon l'une quelconque des revendications précédentes, **caractérisée en ce que** la substance active antisudorale est choisie dans le groupe des sels d'aluminium, de préférence le chlorhydrate d'aluminium et/ou le chlorhydrate d'aluminium activé.

6. Composition selon l'une quelconque des revendications précédentes, **caractérisée en ce que** le rapport chlorhydrate d'aluminium à acide mandélique est choisi dans la plage allant de 15:1 à 1:1, de préférence de 12:1 à 2:1, en particulier de 10:1 à à 2,5:1.

7. Composition selon l'une quelconque des revendications précédentes, **caractérisée en ce que** la substance active antisudorale est utilisée en une quantité de 1 à 35 % en poids, de préférence de 1 à 25 % en poids, façon particulièrement préférée de 1 à 20 % en poids, par rapport à la masse totale de la composition.

8. Composition selon l'une quelconque des revendications précédentes, **caractérisée en ce que** l'acide mandélique est utilisé en une quantité de 0,1 à 10 % en poids, de préférence de 0,1 à 8 % en poids, par rapport à la masse totale de la composition.

9. Composition selon l'une quelconque des revendications précédentes, **caractérisée en ce que** la composition présente une limite d'écoulement définie.

10. Composition selon la revendication 9, **caractérisée en ce que** la composition présente une limite d'écoulement définie de 40 à 120 Pa (à l'aide d'accroissement de contrainte tangentielle en fonction du temps (40 Pa/min ; 25 °C)).

11. Utilisation d'une composition cométique selon au moins l'une des revendications précédentes, pour l'application sur la peau humaine.

12. Utilisation d'une composition cosmétique selon au moins l'une des revendications précédentes 1 à 10, en tant qu'antisudoral et/ou déodorant.

13. Utilisation de l'association d'acide mandélique, d'une substance active antisudorale et/ou d'une substance active déodorante et d'une microémulsion H/E, pour la fabrication d'une préparation antisudorale ou déodorante transparente.

14. Utilisation de l'association d'une substance active antisudorale et/ou d'une substance active déodorante ainsi que d'acide mandélique, pour la préparation d'une microémulsion à limite d'écoulement définie.
